(19) Europäisches Patentamt
European Patent Office
Office européen des brevets

(11) Publication number: **0 010 799**
**B1**

(12) **EUROPEAN PATENT SPECIFICATION**

(45) Date of publication of patent specification: **19.05.82**

(21) Application number: **79200586.0**

(22) Date of filing: **15.10.79**

(51) Int. Cl.³: **C 07 D 209/52,**
C 07 F 7/10, A 01 N 43/90
//(C07D209/52, 221/00)

# (54) A process for the preparation of 3-azabicyclo(3.1.0)hexane derivatives and modifications thereof.

(30) Priority: **27.10.78 GB 4228278**
**14.05.79 GB 7916689**

(43) Date of publication of application:
**14.05.80 Bulletin 80/10**

(45) Publication of the grant of the patent:
**19.05.82 Bulletin 82/20**

(84) Designated Contracting States:
**AT BE CH DE FR GB IT NL SE**

(56) References cited:
GB - A - 1 504 505
US - A - 3 979 204
US - A - 4 047 930
US - A - 4 118 393

Chemical Abstracts, Vol. 70, No. 17, April 28, 1969,
Columbus, Ohio, USA
L. FOWDEN et al.: "Cyclopropane amino acids from Aesculus and Blighia"
page 81, abstract 75105s

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Wood, Derek Alexander**
**76, Sterling Road**
**Sittingbourne, Kent (GB)**
Inventor: **Mason, Ronald Frank**
**"Kingswood"**
**Westwell, Near Ashford Kent (GB)**
Inventor: **Day, Janet Anne**
**3, Palmerstone Walk**
**Sittingbourne, Kent (GB)**
Inventor: **Searle, Robert John Griffith**
**"Highworth" Stockers Hill Rodmersham Green**
**Sittingbourne, Kent (GB)**

(74) Representative: **Rogers, Roy Charles et al,**
**4 York Road**
**London SE1 7NA (GB)**

(56) References cited:
Chemical Abstracts, Vol. 77, No. 3, July 17, 1972,

(continued overleaf)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 010 799 B1

(56) References cited:
Columbus, Ohio, USA
I. ROWLAND et al.: "Inhibition of bacterial growth by cis- and trans-3,4-methano-L-prolines Mechanism of toxicity",
page 110, abstract 14817y

# A process for the preparation of 3-azabicyclo(3.1.0)hexane derivatives and modifications thereof

This invention relates to a process for the preparation of 2-cyano-3-azabicyclo[3.1.0]hexane derivatives and modifications thereof.

2-carboxy-3-azabicyclo[3.1.0]hexane and certain salts and esters thereof have useful biological properties, being capable of sterilising the male anthers in plants. Therefore there is considerable interest in methods for the preparation of these compounds. The available preparation methods, however, have proved to be relatively complex. One possible synthetic route involves the hydrolysis of the corresponding 2-cyano compound, but so far this cyano compound could not be easily synthesised.

European Patent Application No. 79200096 discloses that 2-cyano-3-azabicyclo[3.1.0]hexane and certain substituted derivatives thereof can be prepared by reaction of the corresponding 2-cyano-4-oxo compound with a trialkyl oxoniumfluoroborate and subsequently with a reducing agent. This reaction however is a two-step process which may require the use of expensive reagents.

European Patent Application No. 79200034 discloses that 2-cyano-3-azabicyclo[3.1.0]hexane can be prepared by reacting 3-azabicyclo[3.1.0]hex-2-ene with an alkali metal bisulphite, and subsequently with an alkali metal cyanide. This process too is a two-stage process.

A process has now been found by which a cyano group can be introduced into the bicyclohexane ring system in a single step.

The invention provides a process for the preparation of a compound of the general formula

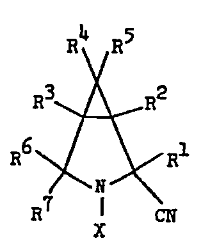

(I)

wherein $R^1$ represents a hydrogen atom or an alkyl group which may be unsubstituted or substituted by one or more alkoxy groups, each of $R^2$, $R^3$, $R^4$, $R^5$, $R^6$ and $R^7$, any two or more of which may be the same or different, represents a hydrogen atom, or an alkyl, aryl, aralkyl or alkaryl group which may be unsubstituted or substituted by one or more alkoxy groups, and X represents a hydrogen atom, an organic acyl group or a trialkylsilyl group; characterised in that a compound of the general formula II and/or the trimer thereof

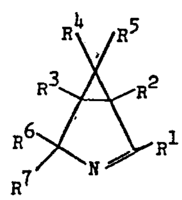

(II)

wherein $R^1$, $R^2$, $R^3$, $R^4$, $R^5$, $R^6$ and $R^7$ have the meanings given for the general formula I, is reacted with a cyanide of the general formula XCN in which X has the meaning given above.

Compounds of the general formula I in which X represents an organic acyl group or a trialkylsilyl group are novel.

Preferably $R^1$ represents a hydrogen atom or an unsubstituted alkyl group of up to 6 carbon atoms; and $R^2$, $R^3$, $R^4$, $R^5$, $R^6$ and $R^7$ each independently represents a hydrogen atom or an unsubstituted alkyl group of up to 6 carbon atoms; or an unsubstituted aryl, alkaryl or aralkyl group of up to 10 carbon atoms.

More preferably, $R^1$ represents a hydrogen atom or an alkyl group of up to 4 carbon atoms, e.g. a methyl group; and $R^2$, $R^3$, $R^4$, $R^5$, $R^6$ and $R^7$ each independently represents a hydrogen atom or an alkyl group of up to 4 carbon atoms, for example a methyl group.

Most preferably, each of $R^1$, $R^2$, $R^3$, $R^4$, $R^5$, $R^6$ and $R^7$ represents a hydrogen atom.

X may for example be an alkanoyl group, for example an acetyl group, or an aroyl group, for example a benzoyl group. In a trialkylsilyl group X, two or three of the alkyl groups may be the same or different, and each alkyl group preferably has up to 4, especially 1 or 2, carbon atoms. An especially preferred trialkylsilyl group is the trimethylsilyl group.

The process according to the invention is suitably carried out in the presence of an inert solvent, for example an alcohol, an ether such as diethyl ether or a hydrocarbon or chlorinated hydrocarbon such

as dichloromethane. A mixture of solvents may be used. The reaction temperature may for example be in the range of from 0 to 60°C, preferably 10 to 30°C. For example, the reaction may be conducted at the reflux temperature of the solvent used. It is however most conveniently conducted at room temperature.

In some cases, it is preferable, and may indeed be essential, to conduct the reaction under moisture-free conditions. This may be the case when using an acyl cyanide or, especially, a trialkylsilyl cyanide as reagent.

The molar ratio of the reactants may vary over a wide range. An excess of the cyanide reactant, for example up to a 5-fold, preferably up to a 3-fold, especially up to a 1.5 fold, excess, may be used. However, it may be desirable to use approximately stoichiometric quantities, especially when the cyanide reactant is benzoyl cyanide, since this may avoid separation problems during work-up.

The cyanide reagent may if desired be prepared and used *in situ*. Trialkylsilyl cyanides may be prepared by the reaction of a trialkylsilyl chloride with potassium cyanide, optionally in the presence of zinc iodide, by the method described in J. Org. Chem. *39* No. 7 (1974) p.916. Whether the resulting trialkylsilyl cyanide is isolated or used *in situ,* it is suitably kept moisture-free.

Hydrogen cyanide for example may be generated *in situ* by the action of a strong mineral acid on an alkali metal cyanide, or by the use of a cyanohydrin under alkaline conditions, but it is preferably added as such, in the form of a gas, a solution in the reaction solvent, or, preferably, a liquid, to the compound of the general formula II and/or the trimer thereof.

The compound of the general formula II and/or its trimer may for example be produced by direct oxidation of a compound of the general formula

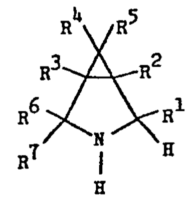

(III)

in which $R^1$—$R^7$ have the meanings given for the general formula I. Manganese dioxide is a suitable reagent, and the oxidation can be performed simply by stirring the compound of the general formula III with manganese dioxide in the presence of a suitable solvent, for example a hydrocarbon such as benzene or light petroleum conveniently at room temperature.

Alternatively, the compound of the general formula II and/or its trimer may be produced by dehydrohalogenation of a compound of the general formula

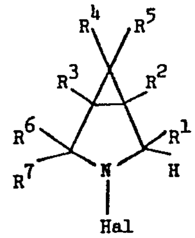

(IV)

wherein each of $R^1$, $R^2$, $R^3$, $R^4$, $R^5$, $R^6$ and $R^7$ has the meaning given for the general formula I, and Hal represents a chlorine or bromine atom.

The dehydrohalogenation may be carried out using any suitable dehydrohalogenating agent, for example a strong organic base such as triethylamine or pyridine in non-aqueous solution, or a strong inorganic base, for example sodium hydroxide, in aqueous or non-aqueous solution. Suitable polar solvents are for example ethers, alcohols, or water. The reaction is preferably carried out at a temperature of up to 150°C, preferably at a temperature in the range 20°C to 80°C. The process can conveniently be carried out at the reflux temperature of the solvent used.

An advantage of carrying out the dehydrohalogenation in a non-aqueous reaction medium is that the resulting solution may if desired by used directly *in situ* in the cyanide addition process according to the invention even if a moisture-sensitive cyanide reagent is used. On the other hand, generally shorter reaction times are required when an aqueous reaction medium is used: in this case however the desired compound may have to be isolated, so that the subsequent cyanide addition can proceed in the absence of moisture.

It is believed that the dehydrohalogenation process leads to a solution consisting largely of the monomer of the general formula II. On removal of the solvent, a solid is produced which has a complex NMR spectrum and is believed to be the trimer:

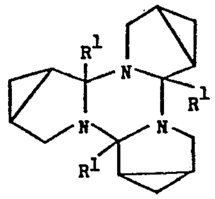

(IIa)

illustrated here with substituents $R^2$ to $R^7$ not shown. When the solid trimer is re-dissolved, a solution is formed in which the trimer is in equilibrium with the monomer, the concentration of each species being dependent on the dilution of the solution. The process according to the invention may be carried out irrespective of the relative proportions of monomer and trimer in the reaction solution.

The compound of the general formula IV may for example be prepared by the N-chlorination or N-bromination of a compound of the general formula III given above. Any suitable chlorinating or brominating agent may be used, for example an N-halo compound, for example N-bromo- or, especially, N-chlorosuccinimide, or an inorganic hypohalite, for example sodium hypochlorite. Sodium hypochlorite may conveniently be used in the form of sodium hydroxide plus chlorine. This process is suitably carried out by admixing a slight excess of the halogenating agent with the compound of the general formula III. Any suitable solvent, for example an ether, may be used. The reaction may for example be carried out at room temperature.

Thus the invention also provides a process for the preparation of a compound of the general formula I, which comprises converting a compound of the general formula III into a compound of the general formula II and/or the trimer thereof, and reacting at least part of said compound with a compound of the formula XCN.

The compounds of the general formula III wherein each of $R^1$, $R^6$ and $R^7$ represents a hydrogen atom may be prepared by reacting a compound of the general formula

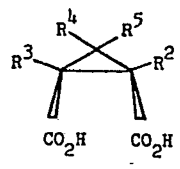

(V)

wherein each of $R^2$, $R^3$, $R^4$ and $R^5$ have meanings given above, or a mono- or di-acyl halide or a mono- or di-ester or the anhydride thereof, with ammonia and optionally water; effecting cyclisation by subjecting the resultant product to elevated temperature to produce a compound of the general formula

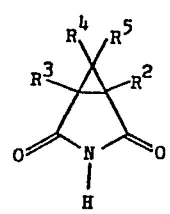

(VI)

wherein each of $R^2$, $R^3$, $R^4$ and $R^5$ have the meanings given above; and reducing the carbonyl groups in the 2 and 4 positions of the compound of the general formula VI, for example using lithium aluminium hydride. Compounds of the general formula IV wherein $R^1$, $R^6$ and/or $R^7$ represent moieties other than hydrogen atoms, may be prepared by introducing these moieties by methods analogous to those known in the art.

A compound of the general formula I may be hydrolysed to form a compound of the general formula

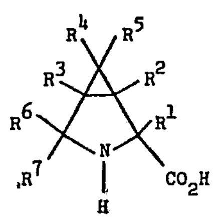

(VII)

in which $R^1$—$R^7$ have the meanings given above. Such compounds have interesting pollen-suppressing and plant growth regulating properties. The hydrolysis may for example be carried out by refluxing the compound of the general formula I in the presence of an aqueous acid. This hydrolysis may lead to an

acid addition salt of the compound of the general formula VII. Refluxing of a compound of the general formula I in the presence of an alcohol and dry hydrogen chloride produces an ester of the compound of the general formula VII or the HCl salt thereof.

Thus, the invention also provides a process for the preparation of a compound of the general formula VII, or a salt and/or ester thereof, which comprises hydrolysing or alcoholising a compound of the general formula I which has been prepared by the process according to the invention.

If X in a compound of the general formula I represents a trialkylsilyl group, it is possible with careful control of the reaction conditions to hydrolyse partially the compound to a compound of the general formula I in which X represents a hydrogen atom. This is generally not possible with a compound of the general formula I in which X represents an acyl group. Mild reaction conditions may be used for the partial hydrolysis, for example the silyl compound may be stirred with water at room temperature.

In the compounds of the general formula I, the 2-cyano group may be cis- or trans- to the $>CR^4R^5$ group, and in addition, for each of these geometric isomers, a pair of optical isomers exists due to the asymmetry of the 2-carbon atom. In addition, other geometric and/or optical isomers may exist depending on the meanings of $R^4$, $R^5$, $R^6$ and $R^7$. In some applications of the process according to the invention, certain isomers are formed exclusively; for example, when each of $R^1$, $R^2$, $R^3$, $R^4$, $R^5$, $R^6$ and $R^7$ represents a hydrogen atom and the cyanide reagent is hydrogen cyanide, the process according to the invention generally leads exclusively to trans 2-cyano-3-azabicyclo[3.1.0]hexane.

As stated above, the compounds of the general formula I may be converted, for example by treatment with strong acid, into the corresponding 2-carboxylic acids. For example trans (D,L) 2-cyano-3-azabicyclo[3.1.0]hexane may be hydrolysed to give trans (D,L) 2-carboxy-3-azabicyclo[3.1.0]hexane. This preparation of a single geometric isomer may be extremely useful since, generally, a mixture of geometric isomers presents manufacturing and formulation problems as the two geometric isomers have different physical properties.

The process according to the invention is of particular interest for the preparation of trans 2-carboxy-3-azabicyclo[3.1.0]hexane, or a salt and/or ester thereof, which may be prepared by hydrolysing or alcoholysing trans 2-cyano-3-azabicyclo[3.1.0]hexane which has been prepared by the reaction of a compound of the formula II and/or the trimer thereof with hydrogen cyanide.

Thus, a preferred route for the preparation of trans 2-carboxy-3-azabicyclo[3.1.0]hexane comprises:

(i) converting 3-azabicyclo[3.1.0]hexane into 3-azabicyclo[3.1.0]hex-2-ene and/or the trimer thereof, for example by direct oxidation or by N-chlorination or N-bromination followed by dehydrohalogenation;

(ii) reacting the product from (i) with hydrogen cyanide to produce trans 2-cyano-3-azabicyclo-[3.1.0]hexane; and

(iii) hydrolysing or alcoholising the product from (ii) to produce 2-carboxy-3-azabicyclo[3.1.0]hexane or a salt and/or ester thereof.

However, in some cases, the direct hydrolysis of a compound of the general formula I containing inorganic impurities, to a free acid of the general formula VII or an acid addition salt thereof, may be rather costly to perform on a large scale. The free acid is an amino acid which is not distillable and is soluble in both acidic and basic solutions. Thus, in order to obtain a product free from inorganic impurities, it is convenient to purify the product by passage over an ion exchange column using a series of aqueous eluants. This procedure is perfectly adequate in the laboratory, but may, on a larger scale, become rather costly in view of the large volumes of demineralised water required for elution of the product.

The need for elution can be avoided by converting the compound of the general formula I obtained by the process according to the invention, into a lower alkyl ester of the acid of the general formula VII, by alcoholysis using the corresponding alkanol in the presence of a non-aqueous acid catalyst, suitably dry hydrogen chloride. Alkanols having 1 to 3 carbon atoms are suitable. The resulting ester is distillable, unlike the free acid, and can therefore be purified by distillation. Subsequently, the ester can be hydrolysed, using water optionally containing a small quantity of a mineral acid, or aqueous ammonia, to produce the free acid. Alkaline hydrolysis is undesirable, since this introduces inorganic ions into the reaction mixture, which need to be removed on an ion exchange column.

If it is required to produce relatively pure trans compounds, the trans 2-cyano compound is preferably isolated or further reacted as soon as possible after its formation.

Thus the invention also provides a process for the preparation of an acid of the general formula VII, which comprises alcoholising a compound of the general formula I which has been prepared by the process according to the invention, using an alkanol having 1 to 3 carbon atoms; purifying the resulting ester by distillation; and hydrolysing the purified ester using water optionally containing a catalytic amount of a mineral acid, or aqueous ammonia, to produce the desired acid. This process is especially advantageous when the compound of the general formula I is trans 2-cyano-3-azabicyclo-[3.1.0]hexane, since it provides a convenient method of preparing trans 2-carboxy-3-azabicyclo[3.1.0]-hexane.

The following Examples illustrate the invention:

Example 1

Preparation of trans 2-cyano-3-azabicyclo[3.1.0]hexane

A slurry of N-chlorosuccinimide (23.6 g, 0.175 mol) in diethyl ether (500 ml) was stirred at room temperature under a nitrogen blanket while 3-azabicyclo 3.1.0 hexane (8.4 g, 0.1 mol) was added. The mixture was stirred at room temperature for 2½ hours under the nitrogen blanket, and was then filtered to remove succinimide. The filtrate was washed with two 100 ml portions of water and one 50 ml portion of a saturated aqueous solution of sodium chloride.

The combined filtrates were dried over $Na_2SO_4$ and concentrated to a volume of approximately 60 ml on a rotary evaporator at 20—25°C and approximately 60 mm Hg pressure. The remaining solution was added to 6.6 g of 85% potassium hydroxide in absolute ethanol (50 ml) at 5—10°C. An exothermic reaction occurred and the temperature rose to 30°C. The mixture was stirred at room temperature overnight under nitrogen, and then filtered. Liquid hydrogen cyanide (5.4 g, 0.2 mol) was added to the filtrate and the temperature rose from 22°C to 29°C over 5 minutes. The mixture was stirred for 1½ hours at room temperature and then stripped on a rotary evaporator at 50°C to give 11 g of an oily product containing some crystals.

Distillation from an oil bath yielded trans 2-cyano-3-azabicyclo[3.1.0]hexane, B.Pt. 56°C at 0.1 mm Hg pressure. Yield: 65%.

Example 2

Preparation of trans 2-carboxy-3-azabicyclo[3.1.0]hexane

A slurry of N-chlorosuccinimide (159 g, 1.2 mol) in diethyl ether (500 ml) was stirred at 20°C whilst 3-azabicyclo[3.1.0]hexane (83 g, 1.0 mol) was added over 30 minutes. The mixture was stirred for a further 1.5 hours, and then filtered to remove the succinimide. The filter pad was washed with two 50 ml portions of diethyl ether and the filtrates were combined.

A solution of sodium hydroxide (48 g, 1.2 mol) in methanol (300 ml) was added to the diethyl ether solution of N-chloro-3-azabicyclo[3.1.0]hexane, and when the initial exothermic reaction had subsided, the mixture was stirred and refluxed overnight (16 hours). After cooling to 20°C, an excess of liquid hydrogen cyanide (40 g) was added to this solution of 3-azabicyclo[3.1.0]hex-2-ene, and the mixture was stirred for 1 hour.

6N hydrochloric acid (800 ml) was added to the resulting solution of trans 2-cyano-3-azabicyclo[3.1.0]hexane, and the reaction mixture was heated at 100°C for 3 hours, during which period the diethyl ether and methanol distilled off, leaving an aqueous acidic solution which was then treated on the H+ form of an ion exchange resin using aqueous ammonia as eluent. Recrystallisation from aqueous isopropyl alcohol gave 77.7 g of a white crystalline material, M.Pt. 248—250°C (with decomposition). Analysis showed this product to be isomerically pure trans (D,L) 2-carboxy-3-azabicyclo[3.1.0]hexane, with a chemical purity of 98.3%. Overall yield from 3-azabicyclo[3.1.0]hexane: 61.2%.

Example 3

Preparation of trans 2-carboxy-3-azabicyclo[3.1.0]hexane under acid conditions

3-azabicyclo[3.1.0]hexane (0.15 mol) was treated with N-chlorosuccinimide and then with potassium hydroxide as in Example 1. The resulting solution was treated with 20 ml 10 N HCl in ethanol. The temperature was held below 10°C by cooling on an ice bath. The resulting mixture was then treated with liquid hydrogen cyanide (7.7 ml, 0.2 mol) at 10—13°C. The reaction mixture was then allowed to reach room temperature and stirred for 2 hours. The pH was approximately 2.

The mixture was then stripped of solvent in a rotary evaporator to give a very viscous orange oil, which was purified by distillation. The resulting product was hydrolysed using 6N hydrochloric acid as in Example 2, to give 2-carboxy-3-azabicyclo[3.1.0]hexane-2-carboxylic acid $^{13}C$ NMR revealed no cis-isomer present.

Example 4

Preparation of trans 2-cyano-3-azabicyclo[3.1.0]hexane under alkaline conditions

The procedure of Example 3 was repeated except that the HCl was replaced by 1.5 ml of triethylamine. The pH of the reaction mixture after addition of hydrogen cyanide was 9—10.

$^{13}C$ NMR showed that the product was trans 2-cyano-3-azabicyclo[3.1.0]hexane, with no cis-isomer present.

Example 5

Preparation of 2-cyano-3-trimethylsilyl-3-azabicyclo[3.1.0]hexane, and its partial hydrolysis

(a) 3-azabicyclo[3.1.0]hex-2-ene and its trimer

A solution of 3-azabicyclo[3.1.0]hexane (8.3 g; 0.1 m) in 50 ml diethyl ether was added to a suspension of N-chlorosuccinimide (23.6 g) in 150 ml diethyl ether at room temperature. The suspension was stirred at room temperature for 2¼ hours then filtered and washed with water. The ethereal solution was dried over $Na_2SO_4$, filtered, and concentrated at 70°C under a vigreaux column to about 25 ml. The residue was added dropwise at 5—10°C to a stirred solution of KOH (6.6 g 85%) in

50 ml methanol. The suspension was stirred at 0—5°C for 2 hours then left overnight at room temperature. The mixture was concentrated at 35°C and a pressure of 18 cm Hg for 6 hours then water (50 ml) was added. The mixture was continuously extracted with diethyl ether.

The diethyl ether was distilled off at atmospheric pressure then the residue was distilled at atmospheric pressure. The first fraction obtained, boiling point 77—96°C, was 3 g of a colourless liquid shown by infra-red and NMR spectra to be an aqueous solution of the monomer 3-azabicyclo[3.1.0]hex-2-ene.

The second fraction boiling at 96—110°C, condensed to give 3.8 g of an off-white solid, melting point 60—63°C which was shown by infra-red and NMR spectroscopy to be the trimer of this imine.

Analysis of white solid:

| | C | H | N |
|---|---|---|---|
| Calculated for $C_{15}N_3H_{21}$ | 73.2 | 9.1 | 17.2 |
| Found | 74.0 | 8.7 | 17.3 |

(b) 2-cyano-3-trimethylsilyl-3-azabicyclo[3.1.0]hexane and its partial hydrolysis

Trimethylsilyl cyanide (16 ml; 0.128 m) was added to a stirred solution of the imine trimer prepared in (a) above (7 g; 28.8 mm) in 700 ml dry $CH_2Cl_2$ at room temperature under nitrogen with exclusion of moisture. The solution was stirred at room temperature for 24 hours, to give a solution of 2-cyano-3-trimethylsilyl-3-azabicyclo[3.1.0]hexane.

The solution was poured onto 1 litre of water, stirred at room temperature for 30 minutes, and extracted with $CH_2Cl_2$. The extract was dried over $Na_2SO_4$ and evaporated down to give 9.4 g of a yellow liquid.

This liquid was purified by distillation and the fraction boiling at 72—74°C under a pressure of 0.7 mm Hg (5.45 g) was identified by NMR as 2-cyano-3-azabicyclo[3.1.0]hexane.

Example 6

Preparation of 2-cyano-3-benzoyl-3-azabicyclo[3.1.0]hexane and its hydrolysis

(a) Addition of benzoyl cyanide to an imine trimer

Benzoyl cyanide (11.3 g; 86.4 mMol) was added to a solution of the imine trimer prepared as in Example 5 (a) above (7 g; 28.8 mm) in 700 ml dry $CH_2Cl_2$. The solution was stirred at room temperature overnight and then evaporated down. The resulting oil was distilled at a temperature of 160°C and a pressure of 0.1 mm Hg, and the distillate was treated with petrol/ether and filtered.

The resulting compound 4.8 g of a solid having a melting point of 74—77°C, was identified by infra-red and NMR spectra as 2-cyano-3-benzoyl-3-azabicyclo[3.1.0]hexane. The spectra data are as follows:

| | | |
|---|---|---|
| IR: | 1650 $cm^{-1}$: | C = O Stretch |
| | 2240 $cm^{-1}$: | C = N Stretch |
| NMR: | 0.2 ppm | 1H (multiplet) |
| | 0.7 ppm | 1H (multiplet) |
| | 1.7 ppm | 2H (multiplet) |
| | 3.6 ppm | 2H (multiplet) |
| | 5.1 ppm | 1H (multiplet) |
| | 7.3 ppm | 5H (singlet) |

(b) Preparation of 2-carboxy-3-azabicyclo[3.1.0]hexane

A solution of the compound prepared in (a) above (1 g) in 50 ml $CH_2CL_2$ was stirred and heated under reflux overnight. The suspension was cooled, washed with diethyl ether, and the aqueous layer evaporated to dryness. The residue was dissolved in water and percolated through an acidic ionexchange column "DOWEX 50" (Trade Mark). The ion exchange bed was washed with water and the product was eluted with $2NNH_4OH$.

0.4 g of a product was obtained. NMR showed that this product was 2-carboxy-3-azabicyclo[3.1.0]hexane, containing cis and trans isomers in 1:1 ratio.

Example 7

Preparation of trans 2-carboxy-3-azabicyclo[3.1.0]hexane via the corresponding ethyl ester

N-chlorosuccinimide (4.01 kg, 30 mol) was suspended in 12.5 l diethyl ether, and 3-azabicyclo[3.1.0]hexane (2.08 kg, 25 mol) was added over 80 minutes. The temperature was held within the range 11 to 22°C during the addition and then within the range 18 to 20 for a further 2 hours. The reaction mixture was then filtered, and added over 2 hours to a solution of sodium hydroxide (1.2 kg. 30 M) in ethanol (10 l) under a blanket of nitrogen and at a temperature of 10°C. The

temperature rose to 23°C, and then over the next hour to 38°C. the mixture was then refluxed for 6 hours, and cooled to 10°C.

Liquid hydrogen cyanide (844 g, 31.25 mol) was added over 30 minutes and stirring was continued at room temperature for a further hour. The resulting mixture was filtered, and then added over 20 minutes to 22.5 l absolute ethanol containing 7.3 kg (200 mol) anhydrous hydrogen chloride maintained at a temperature between −10 and +2°C. At this stage a further batch of trans 2-cyano-3-azabicyclo[3.1.0]hexane solution, prepared from 50 mol 3-azabicyclo[3.1.0]hexane, was also added to the reaction mixture. Ether was then distilled off, and the mixture was refluxed for 4 hours and then cooled to −5°C. Anhydrous ammonia was passed into the reaction mixture for 2 hours, until the pH was 10.0.

The reaction mixture was filtered, and the solvent was stripped on a rotary evaporator.

There were obtained 5.42 kg of 2-ethoxycarbonyl-3-azabicyclo[3.1.0]hexane, which was shown by NMR to be chemically 99% pure and to contain 96% trans isomer. This represents an overall yield of 70% based on 3-azabicyclo[3.1.0]hexane.

This preparation was repeated several times. 8.3 kg (53.3 mol) of the prepared trans 2-ethoxycarbonyl-3-azabicyclo[3.1.0]hexane were added over 1 hour to 217 mls of concentrated hydrochloric acid in 83 l water, under reflux. Heating was continued for a further 2½ hours during which time ethanol was distilled off. Water was then stripped from the reaction mixture in a rotary evaporator until crystals formed. Isopropyl alcohol was added, and the resulting precipitate was filtered and the solid residue was washed with isopropyl alcohol and then hexane and air dried.

The product was 5.08 kg (40 mol) 2-carboxy-3-azabicyclo[3.1.0] hexane, 98% of which was the trans isomer. This was a yield of 75% based on the corresponding ethyl ester.

A further crop of 1.20 kg of 2-carboxy-3-azabicyclo[3.1.0]hexane was obtained from the washings and filtrate. This crop had a trans: cis ratio of 33.65.

## Example 8

Preparation of 2-cyano-3-azabicyclo[3.1.0]hexane via oxidation of 3-azabicyclo[3.1.0]hexane

A mixture of 3-azabicyclo[3.1.0]hexane (1 g 0.012 mol), manganese dioxide (5 g, 0.057 mol) and petrol having a boiling point range of 40—60°C, was stirred at room temperature for 4 hours. Sodium sulphate was added and the mixture was allowed to stand for a further 4 hours, and then filtered. Thin layer chromatography showed the presence of the trimer of 3-azabicyclo[3.1.0]hex-2-ene.

Trimethylsilyl cyanide (3 ml, 0.024 mol) was then added, and the mixture was stirred under nitrogen at room temperature for 20 hours. The solution became cloudy, and some solid precipitated. 10 ml water were added and the mixture was stirred for 1 hour. The aqueous layer was separated and washed with diethyl ether, and the combined organic phases were dried over sodium sulphate, filtered and evaporated down. 1.05 g of a straw coloured oil were obtained. 80% of this product was 2-cyano-3-azabicyclo[3.1.0]hexane, and 20% was unreacted 3-azabicyclo[3.1.0]hexane.

## Claims

1. A process for the preparation of a compound of the general formula

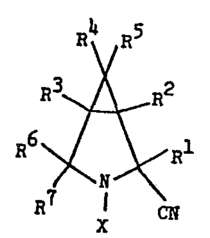

(I)

wherein $R^1$ represents a hydrogen atom or an alkyl group which may be unsubstituted or substituted by one or more alkoxy groups, each of $R^2$, $R^3$, $R^4$, $R^5$, $R^6$ and $R^7$, any two or more of which may be the same or different, represents a hydrogen atom, or an alkyl, aryl, aralkyl, or alkaryl group which may be unsubstituted or substituted by one or more alkoxy groups, and X represents a hydrogen atom, an organic acyl group or a trialkylsilyl group; characterised in that a compound of the general formula II and/or the trimer thereof:

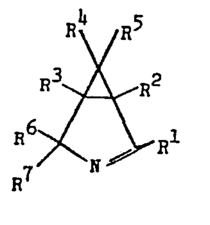

(II)

wherein $R^1$, $R^2$, $R^3$, $R^4$, $R^5$, $R^6$ and $R^7$ have the meanings given for the general formula I, is reacted with a cyanide of the general formula XCN in which X has the meaning given above.

2. A process as claimed in Claim 1, characterised in that a starting material is used in which each of $R^1$ to $R^7$ represents a hydrogen atom or an alkyl group having up to 4 carbon atoms.

3. A process as claimed in Claim 2, characterised in that a starting material is used in which each of $R^1$ to $R^7$ represents a hydrogen atom.

4. A process as claimed in any one of Claims 1 to 3, characterised in that a cyanide reactant is used in which X represents a hydrogen atom, an alkanoyl or aroyl group or a trialkylsilyl group having up to 4 carbon atoms in each alkyl group.

5. A process as claimed in Claim 4, characterised in that a cyanide reactant is used in which X represents a hydrogen atom, a benzoyl group or a trimethylsilyl group.

6. A process as claimed in Claim 5, characterised in that the cyanide reactant is hydrogen cyanide.

7. A process as claimed in any one of Claims 1 to 6, characterised in that the reaction temperature is in the range of from 0 to 60°C.

8. A process as claimed in Claim 7, characterised in that the reaction temperature is in the range of from 10 to 30°C.

9. A process as claimed in any one of Claims 1 to 8, characterised in that an excess of the cyanide reactant of up to 5 fold is used.

10. A process as claimed in any one of Claims 1 to 9, characterised in that there is used as starting material a compound of the general formula II and/or the trimer thereof which has been prepared from a compound of the general formula

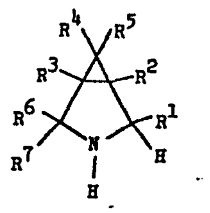

(III)

in which $R^1$—$R^7$ have the meanings given for the general formula I.

11. A process as claimed in Claim 10, characterised in that the compound of the general formula III has been directly oxidised to the compound of the general formula II and/or the trimer thereof, or has been N-chlorinated or N-brominated and subsequently dehydrohalogenated to give the compound of the general formula II and/or the trimer thereof.

12. A process as claimed in any one of Claims 1 to 11, characterised in that it also comprises a further step in which the compound of the general formula I is hydrolysed and/or alcoholised to produce a compound of the general formula VII or a salt and/or an ester thereof:

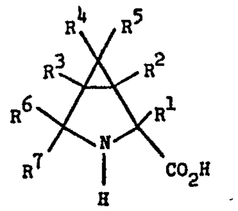

(VII)

in which $R^1$ to $R^7$ have the meanings given for the general formula I.

13. A process as claimed in Claim 12, characterised in that hydrogen cyanide is added to 3-azabicyclo[3.1.0]hex-2-ene and/or the trimer thereof to produce trans 2-cyano-3-azabicyclo[3.1.0]-hexane, which compound is hydrolysed and/or alcoholised to produce trans 2-carboxy-3-azabicyclo-[3.1.0]hexane or a salt and/or an ester thereof.

14. A process as claimed in either Claim 12 or Claim 13, characterised in that the compound of the general formula I is alcoholised using an alkanol having 1 to 3 carbon atoms, the resulting ester is purified by distillation; and the purified ester is hydrolysed using water optionally containing a catalytic amount of a mineral acid, or aqueous ammonia, to produce an acid of the general formula VII given in Claim 12.

**Revendications**

1. Procédé pour la préparation d'un composé de la formule générale:

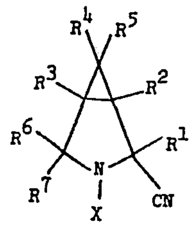

(I)

dans laquelle $R^1$ représente un atome d'hydrogène ou un groupe alcoyle qui peut être non-substitué ou substitué par un ou plusieurs groupes alcoxy, chacun des substituants $R^2$, $R^3$, $R^4$, $R^5$, $R^6$ et $R^7$, dont deux quelconques ou plus peuvent être identiques ou différents, représente un atome d'hydrogène ou un groupe alcoyle, aryle, aralcoyle ou alcaryle qui peut être nonsubstitué ou substitué par un ou plusieurs groupes alcoxy, et X représente un atome d'hydrogène, un groupe acyle organique ou un groupe trialcoylsilyle, caractérisé en ce qu'un composé de la formule générale II et/ou son trimère:

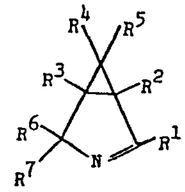

(II)

où $R^1$, $R^2$, $R^3$, $R^4$, $R^5$, $R^6$ et $R^7$ ont les significations indiquées pour la formule générale I, est mis à réagir avec un cyanure de la formule générale XCN dans laquelle X a la signification indiquée ci-dessus.

2. Procédé selon la revendication 1, caractérisé en ce qu'on utilise une matière de départ dans laquelle chacun des substituants $R^1$ à $R^7$ représente un atome d'hydrogène ou un groupe alcoyle ayant jusqu'à 4 atomes de carbone.

3. Procédé selon la revendication 2, caractérisé en ce qu'on utilise une matière de départ dans laquelle les substituants $R^1$ à $R^7$ sont chacun une atome d'hydrogène.

4. Procédé selon l'une quelconque des revendications 1 à 3, caractérisé en ce qu'on utilise un corps en réaction cyanure dans lequel X représente un atome d'hydrogène, un groupe alcanoyle ou aroyle ou un groupe trialcoylsilyle ayant jusqu'à 4 atomes de carbone dans chaque groupe alcoyle.

5. Procédé selon la revendication 4, caractérisé en ce qu'on utilise un corps en réaction cyanure dans lequel X représente un atome d'hydrogène, un groupe benzoyle ou un groupe triméthylsilyle.

6. Procédé selon la revendication 5, caractérisé en ce que le corps en réaction cyanure est l'acide cyanhydrique.

7. Procédé selon l'une quelconque des revendications 1 à 6, caractérisé en ce que la température de réaction est comprise entre 0 et 60°C.

8. Procédé selon la revendication 7, caractérisé en ce que la température de réaction est comprise entre 10 et 30°C.

9. Procédé selon l'une quelconque des revendications 1 à 8, caractérisé en ce qu'on utilise un excès du corps en réaction cyanure allant jusqu'à une quantité quintuple.

10. Procédé selon l'une quelconque des revendications 1 à 9, caractérisé en ce qu'on utilise comme matière de départ un composé de la formule générale II et/ou son trimère ayant été préparés à partir d'un composé de la formule générale:

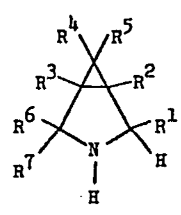

III

dans laquelle $R^1$ à $R^7$ ont les significations indiquées pour la formule générale I.

11. Procédé selon la revendication 10, caractérisé en ce que le composé de la formule générale III a été oxydé directement pour donner le composé de la formule générale II et/ou son trimère, ou a été N-chloré ou N-bromé et ensuite déshalogénhydraté pour donner le composé de la formule générale II et/ou son trimère.

12. Procédé selon l'une quelconque des revendications 1 à 11, caractérisé en ce qu'il comprend aussi l'étape supplémentaire dans laquelle le composé de la formule générale I est hydrolysé et/ou alcoolysé pour produire un composé de la formule générale VII ou un sel et/ou ester de ce composé:

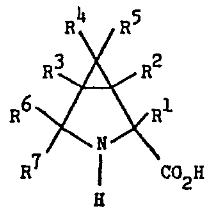

(VII)

où $R^1$ à $R^7$ ont les significations indiquées pour la formule générale I.

13. Procédé selon la revendication 12, caractérisé en ce qu'on ajoute de l'acide cyanhydrique à du 3-azabicyclo(3.1.0)-hex-2-ène et/ou à son trimère de manière à produire du trans 2-cyano-3-azabicyclo(3.1.0)hexane, lequel composé est hydrolysé et/ou alcoolysé pour produire du trans 2-carboxy-3-azabicyclo(3.1.0)hexane ou un sel et/ou un ester de ce composé.

14. Procédé selon l'une des revendications 12 et 13, caractérisé en ce qu'on alcoolyse le composé de la formule générale I en utilisant un alcanol ayant de 1 à 3 atomes de carbone, l'ester résultant est purifié par distillation et on hydrolyse l'ester purifié en utilisant de l'eau contenant éventuellement une quantité catalytique d'un acide minéral, ou de l'ammoniac aqueux, de manière à produire un acide de la formule générale VII donnée dans la revendication 12.

**Patentansprüche**

1. Verfahren zur Herstellung einer Verbindung der allgemeinen Formel:

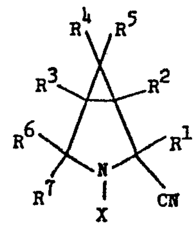

(I)

worin $R^1$ ein Wasserstoffatom oder eine Alkylgruppe bedeutet, die unsubstituiert oder durch eine oder mehrere Alkoxygruppen substituiert sein kann, jeder der Reste $R^2$, $R^3$, $R^4$, $R^5$, $R^6$ und $R^7$, von denen zwei oder mehrere beliebige gleich oder verschieden sein können, für ein Wasserstoffatom oder für eine Alkyl-, Aryl, Aralkyl- oder Alkarylgruppe, die unsubstituiert oder durch eine oder mehrere Alkoxy-, gruppen substituiert sein können, steht und X ein Wasserstoffatom, eine organische Acylgruppe oder eine Trialkylsilylgruppe bedeutet, dadurch gekennzeichnet, dass eine Verbindung der allgemeinen Formel:

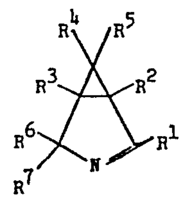

(II)

und/oder das Trimer hiervon, in welcher Formel $R^1$, $R^2$, $R^3$, $R^4$, $R^5$, $R^6$ und $R^7$ die vorstehend für die allgemeine Formel (I) angegebene Bedeutung haben, mit einem Cyanid der allgemeinen Formel XCN, worin X die vorstehend angegebene Bedeutung hat, umgesetzt wird.

2. Verfahren nach Anspruch 1, dadurch gekennzeichnet, dass ein Ausgangsmaterial verwendet wird, worin jeder der Reste $R^1$ bis $R^7$ ein Wasserstoffatom oder eine Alkylgruppe mit bis zu 4 Kohlenstoffatomen bedeutet.

3. Verfahren nach Anspruch 2, dadurch gekennzeichnet, dass ein Ausgangsmaterial verwendet wird, worin jeder der Reste $R^1$ bis $R^7$ ein Wasserstoffatom bedeutet.

4. Verfahren nach einem der Ansprüche 1 bis 3, dadurch gekennzeichnet, dass eine Cyanid-Reaktionskomponente verwendet wird, worin X ein Wasserstoffatom, eine Alkanoyl- oder Aroylgruppe oder eine Trialkylsilylgruppe mit bis zu 4 Kohlenstoffatomen in jeder Alkylgruppe bedeutet.

5. Verfahren nach Anspruch 4, dadurch gekennzeichnet, dass eine Cyanid-Reaktionskomponente verwendet wird, worin X ein Wasserstoffatom, eine Benzoylgruppe oder eine Trimethylsilylgruppe darstellt.

6. Verfahren nach Anspruch 5, dadurch gekennzeichnet, dass die Cyanid-Reaktionskomponente Cyanwasserstoff ist.

7. Verfahren nach einem der Ansprüche 1 bis 6, dadurch gekennzeichnet, dass die Reaktionstemperatur in dem Bereich von 0 bis 60°C liegt.

8. Verfahren nach Anspruch 7, dadurch gekennzeichnet, dass die Reaktionstemperatur in dem Bereich von 10 bis 30°C liegt.

9. Verfahren nach einem der Ansprüche 1 bis 8, dadurch gekennzeichnet, dass eine Überschuss an der Cyanid-Reaktionskomponente in bis zu 5-fachem Ausmass angewendet wird.

10. Verfahren nach einem der Ansprüche 1 bis 9, dadurch gekennzeichnet, dass als Ausgangsmaterial eine Verbindung der allgemeinen Formel (II) und/oder dessen Trimer eingesetzt wird, die bzw. das aus einer Verbindung der allgemeinen Formel:

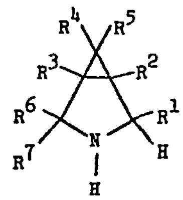

(III)

worin die Reste $R^1$ bis $R^7$ die zur allgemeinen Formel (I) angegebene Bedeutung haben, hergestellt worden ist.

11. Verfahren nach Anspruch 10, dadurch gekennzeichnet, dass die Verbindung der allgemeinen Formel (III) direkt zur Verbindung der allgemeinen Formel (II) und/oder zum Trimer hiervon oxidiert worden ist oder N-chloriert oder N-bromiert und hierauf dehydrohalogeniert worden ist, um die Verbindung der allgemeinen Formel (II) und/oder das Trimer hiervon zu erhalten.

12. Verfahren nach einem der Ansprüche 1 bis 11, dadurch gekennzeichnet, dass es auch eine weitere Stufe umfasst, in welcher die Verbindung der allgemeinen Formel (I) hydrolysiert und/oder alkoholysiert wird, um eine Verbindung der allgemeinen Formel:

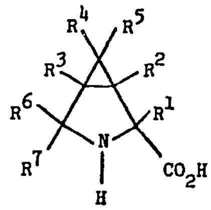

(VII)

oder ein Salz und/oder ein Ester hiervon zu ergeben, in welcher Formel die Reste $R^1$ bis $R^7$ die zur allgemeinen Formel (I) angegebene Bedeutung besitzen.

13. Verfahren nach Anspruch 12, dadurch gekennzeichnet, dass zu 3-Azabicyclo[3.1.0]hex-2-en und/oder dem Trimer hiervon Cyanwasserstoff zugesetzt wird, um trans-2-Cyano-3-azabicyclo[3.1.0]-hexan zu erhalten, welche Verbindung hydrolysiert und/oder alkoholysiert wird, um trans-2Carboxy-3-azabicyclo[3.1.0]hexan oder ein Salz und/oder einen Ester hiervon zu erhalten.

14. Verfahren nach Anspruch 12 oder 13, dadurch gekennzeichnet, dass die Verbindung der allgemeinen Formel (I) unter Verwendung eines Alkanols mit 1 bis 3 Kohlenstoffatomen alkoholysiert wird, der gebildete Ester durch Destillation gereinigt wird, und der gereinigte Ester hydrolysiert wird, wobei Wasser, das gegebenenfalls eine katalytische Menge Mineralsäure enthält, oder wässriges Ammoniak verwendet wird, um eine Säure der allgemeinen Formel (VII), wie sie in Anspruch 12 angegeben ist, zu erhalten.